(19) Europäisches Patentamt
European Patent Office
Office européen des brevets

(11) EP 2 735 329 A2

(12) EUROPEAN PATENT APPLICATION

(43) Date of publication:
28.05.2014 Bulletin 2014/22

(51) Int Cl.:
A61M 16/00 (2006.01)    A61M 16/20 (2006.01)

(21) Application number: 13193808.6

(22) Date of filing: 21.11.2013

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 26.11.2012 MY PI2012005095

(71) Applicant: Hill-Rom Services Pte. Ltd.
Singapore 768923 (SG)

(72) Inventors:
• Toh, Beng Leong
643648 Singapore (SG)

• Lim, Eng Chuan
540613 Singapore (SG)
• Tan, Hee Choon
754589 Singapore (SG)
• Changguo, Mike Yang
Singapore (SG)
• Jee, Soo Yao
544122 Singapore (SG)

(74) Representative: Findlay, Alice Rosemary
Reddie & Grose LLP
16 Theobalds Road
London WC1X 8PL (GB)

(54) **Pulse generator systems for therapy device**

(57) A pulse generator (18) for use with a system (10) for mobilizing lung secretions comprising: a generator body (44) ; a plunger (26) disposed inside the body (44), the plunger (26) having a first end and a second end and being moveable to regulate fluid flow between a plunger port (24) and an outlet port (40); a diaphragm assembly (106) having an input side and an output side; a first diaphragm (28) between the second end of the plunger (26) and the diaphragm assembly (106), the first diaphragm (28) having a plunger side facing the plunger (26) and an opposite side facing the diaphragm assembly (106); a fluid channel extending from the outlet port (40) to the input side of the diaphragm assembly (106); an orifice (38) and a valve (36) residing in the fluid channel.

FIG. 1

**Description**

Mobilization of lung secretions in patients with certain health conditions is an ongoing challenge. While several systems and methods are available for mobilization of lung secretion an opportunity exists for continued development in this area.

The present disclosure includes one or more of the features recited in the appended claims and/or the following features which, alone or in any combination, may comprise patentable subject matter.

One embodiment of a system for mobilizing lung secretions comprises a controller configured to allow selection of continuous oscillation therapy. A pulse generator may be configured to provide a pulsed flow of compressed gas upon selection of said continuous oscillation therapy. A circuit may be configured to deliver said pulsed flow of compressed gas to a person.

One embodiment of a pulse generator for use with a system for mobilizing lung secretions may comprise a body configured to receive compressed supply gas via a first port. A valve stem may be configured to be acted upon by said compressed supply gas and may be housed in said body. A first diaphragm may be configured to support said valve stem, the first diaphragm configured to deform upon application of force by the valve stem allowing a fluidic connection to be established between the first port and a second port of the body.

One embodiment of an airway clearance system may comprise a body comprising means to supply oscillating pressure gas from a first port. An orifice may be configured to receive at least a portion of the oscillating pressure gas from the second port, the orifice may be external to the body; the orifice may be configured to supply gas to a second port of said body.

One method of providing oscillatory pressurized gas for therapy may comprise receiving pressurized gas at a first port of a body of a pulse generator. Supply pressurized gas received from said first port of said body to a second port of said body by displacing a valve stem upon action of pressurized gas to allow fluidic communication between said first port and said second port. Supplying pressurized gas from said second port to an orifice wherein said orifice is configured to supply pressurized gas at a lower pressure than pressure of gas it receives, the orifice external to the body and supplying gas from the orifice to a third port of the body.

The invention will now be further described by way of example with reference to the accompanying drawings, in which:

FIG. 1 is a perspective view of one embodiment of a system to mobilize lung secretions, constructed according to one or more of the principles disclosed herein;

FIG. 2 is a schematic of a pulse generator for use with a system to mobilize lung secretions, constructed according to one or more of the principles disclosed herein;

FIGS. 3 - 6 are schematics showing operation of the pulse generator of FIG. 2 for use with a system to mobilize lung secretions, constructed according to one or more of the principles disclosed herein;

FIG. 7 is an exploded view of a pulse generator for use with a system to mobilize lung secretions, constructed according to one or more of the principles disclosed herein;

FIG. 8A & FIG. 8B are perspective and front cross sectional views respectively of one embodiment of a pulse generator for use with a system to mobilize lung secretions, constructed according to one or more of the principles disclosed herein.

It should be noted that the features illustrated in the drawings are not necessarily drawn to scale, and features of one embodiment may be employed with other embodiments as the skilled artisan would recognize, even if not explicitly stated herein. Descriptions of well-known components and processing techniques may be briefly mentioned or omitted so as to not unnecessarily obscure the described embodiments. The examples used herein are intended merely to facilitate an understanding of ways in which the embodiments may be practiced and to further enable those of skill in the art to practice the embodiments. Accordingly, the examples and embodiments herein are merely illustrative. Moreover, it is noted that like reference numerals represent similar parts throughout the several views of the drawings.

It is understood that the subject matter disclosed is not limited to the particular methodology, protocols, devices, apparatus, materials, applications, etc., described herein, as these may vary. It is also to be understood that the terminology used herein is used for the purpose of describing particular embodiments only.

Unless defined otherwise, all technical and scientific terms used herein have the same meanings as commonly understood by one of ordinary skill in the art.

The subject matter herein is directed to systems and methods for providing a pneumatic form of chest physiotherapy, specifically continuous high frequency oscillation therapy.

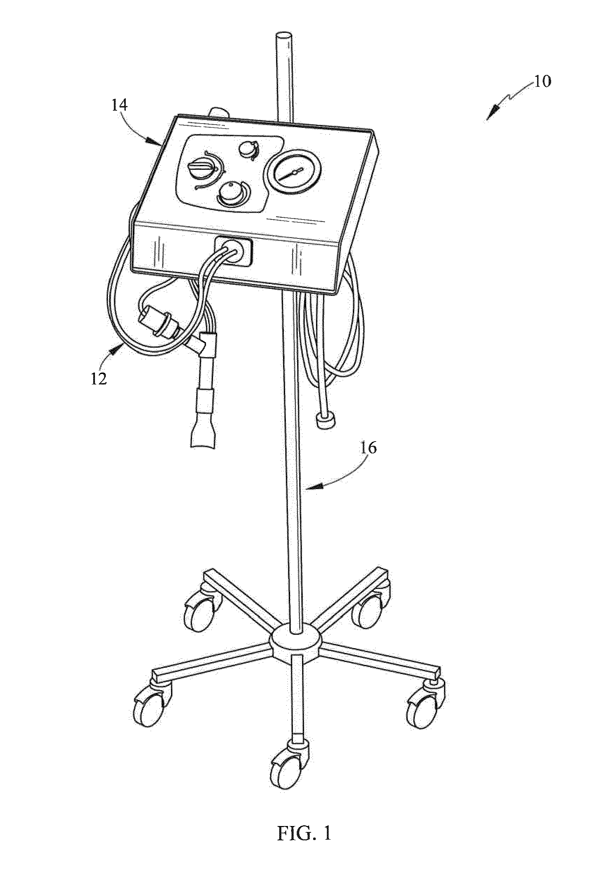

A system 10 for mobilizing lung secretions is shown in FIG. 1. The system 10 shown in FIG. 1 may also be used for lung expansion therapy and / or prevention of pulmonary atelectasis and / or providing supplemental oxygen when used with compressed oxygen in other embodiments. As shown in FIG. 1 the system 10 comprises a circuit 12 which comprises a mouthpiece, handset, nebulizer, tubing and bio-filter / tri-connector in this embodiment. The system 10 also comprises a controller 14 which allows for selection between an aerosol delivery only mode, a continuous high frequency oscillation (CHFO) mode and a Continuous positive expiratory pressure (CPEP) mode. The system 10 also comprises a stand 16 which in this embodiment includes wheels for transport. In other embodiments components of the system 10 for mobilizing secretions may be optional and / or other components included, such permutations are contemplated to be part of the subject matter herein. In this embodiment the CPEP mode comprises providing medicated aerosol combined with continuous positive pressure to assist in holding open and expanding the airways. In one exemplary embodiment the system 10 is The MetaNeb® System.

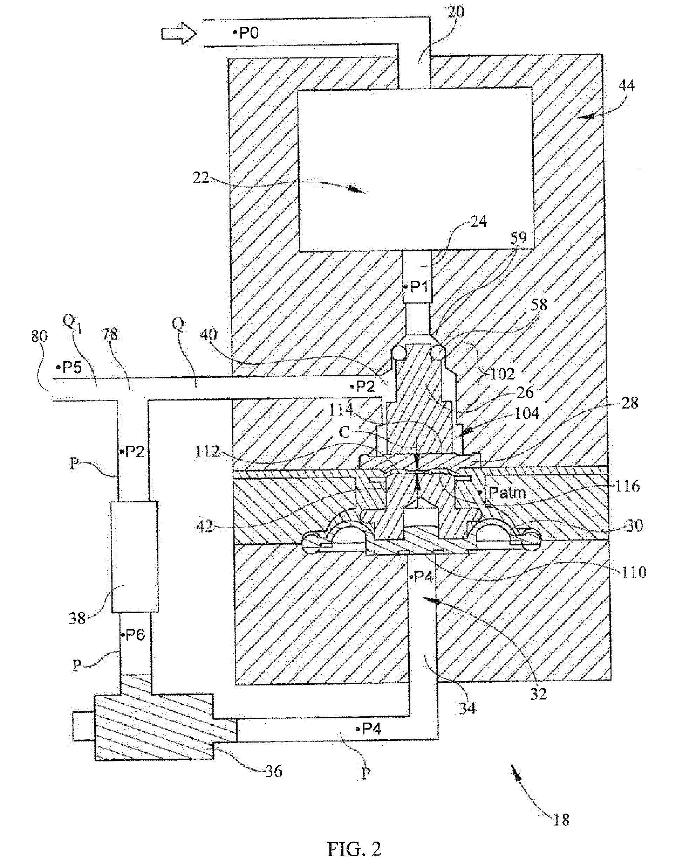

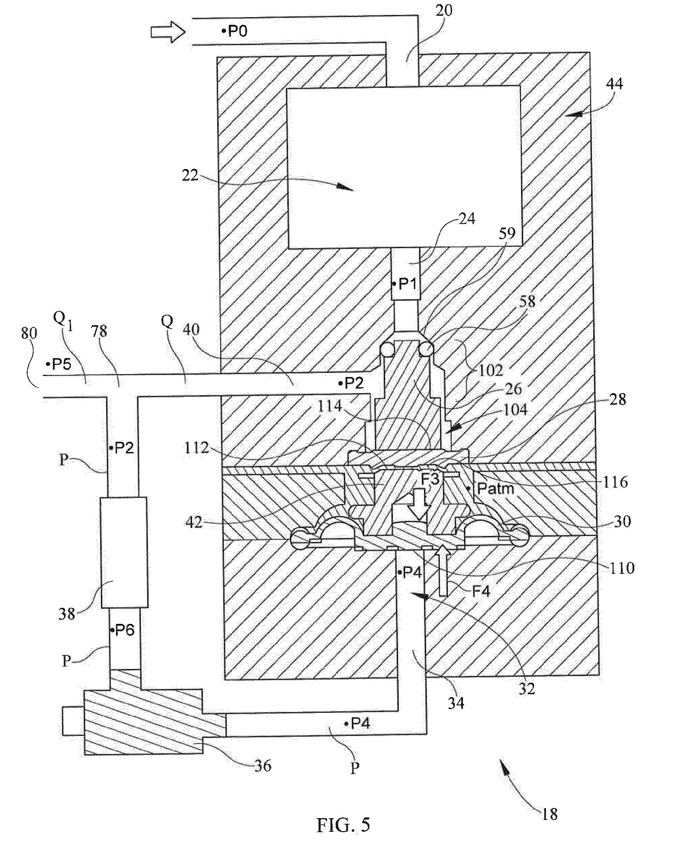

Continuous high frequency oscillation (CHFO) mode in this embodiment allows a pneumatic form of chest physiotherapy that delivers medicated aerosol while oscillating the airways with continuous pulses of positive pressure. In the embodiment described herein a pulse generator 18 (shown in FIGS. 2 - 8B) provides continuous pulses of positive pressure during both inspiration and expiration and is a mechanical system. In other embodiments any combination of electro-mechanical devices and / or electronics including but not limited to actuators, pumps, blowers, fans and electronic circuits may be incorporated in the system 10 to mobilize secretions in the lungs. In this embodiment pulse generator 18 is housed within the controller 14 while in other embodiments the pulse generator 18 is mounted to the stand 16 or while in another embodiment is a standalone unit. The pulse generator 18 configured to communicate with the controller 14 and circuit 12.

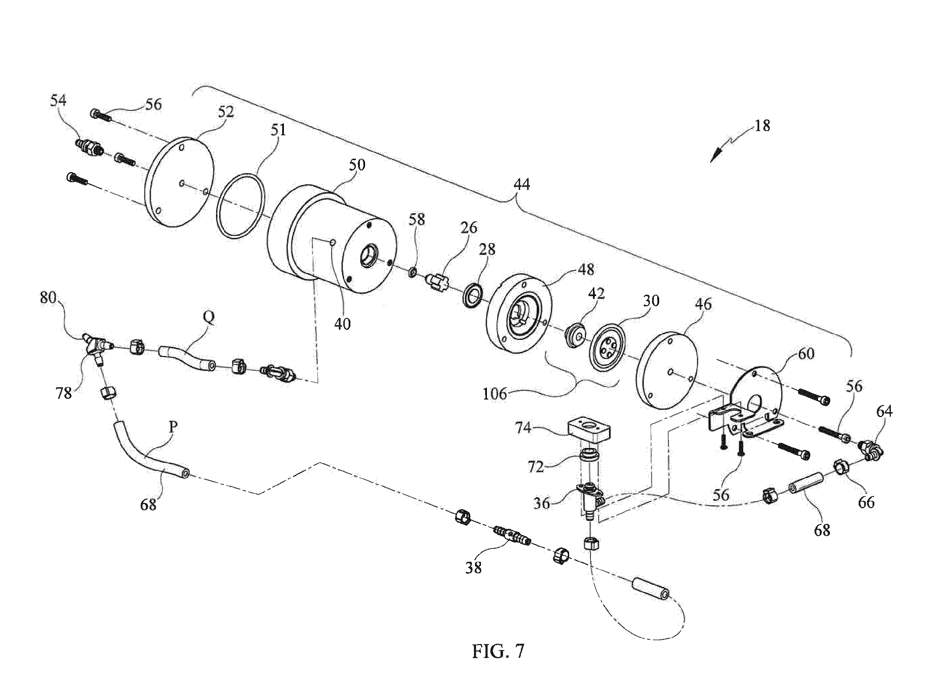

FIG. 2 shows a schematic of a pulse generator 18 for use with a system 10 to mobilize lung secretions. The pulse generator 18 comprises a body 44 which receives compressed gas through inlet port 20 (also referred to as first port) shown in FIG. 2. In this embodiment the compressed gas is air while in other embodiments the compressed gas is oxygen, in yet another embodiment any combination of gases may be supplied to the pulse generator 18. In this embodiment the body 44 comprises an air volume chamber (AVC) 22 (also referred to as first chamber 22) configured to receive air from inlet port 20. The AVC 22 is configured to stabilize the pressure and make up for line losses. As shown in FIG. 2 gases flow from the AVC 22 through plunger port 24 (also referred to as an intermediate port) and apply pressure on valve stem or plunger 26 with pressure P1. The plunger has a first end 102 and a second end 104. The valve stem 26 includes an O-ring 58. P1 acts on area A1 which is the top surface area of the valve stem. The valve stem 26 is configured to obscure fluidic connection between plunger port 24 and outlet port 40 (also referred to as second port). The pulse generator includes a first diaphragm 28 and a second diaphragm 30. As seen best in FIG. 7 a diaphragm assembly 106 is comprised of the second diaphragm 30 and a button 42. The first diaphragm has a plunger side 114 facing the plunger and an opposite side 116 facing the diaphragm assembly 106. The diaphragm assembly has an input side 110 and an output side 112. The valve stem 26 is configured to apply a component of the force it experiences due to pressure P1 on to first diaphragm 28. In this embodiment first diaphragm 28 is made of an elastomeric material while in other embodiment the first diaphragm 28 may be made of any material including but not limited to spring steel. In other embodiments the diaphragm may be of any shape including but not limited to a helical structure. A valve button 42 is configured to be separated from first diaphragm 28 in one embodiment by a clearance space C. In other embodiments the valve button 42 is in contact with first diaphragm 28. Motion of second diaphragm 30 is configured to move valve button 42. Gases from outlet port 40 are configured to feed into a flow restricting orifice 38 and also supply pressurized gas to a patient by way of discharge leg 80 of Wye fitting 78. In this embodiment the orifice 38 is a nozzle. Gases entering orifice 38 at pressure P2 exit the orifice 38 at pressure P6 which is less than P2. In this embodiment the gases exiting the orifice 38 enter a needle valve 36 and therefrom apply pressure P4 on second diaphragm 30 via second chamber 32. In this embodiment the needle valve 36 is configured to be situated outside the body 44 minimizing potential for thermal deformation of the body 44 and / or other components affecting the needle valve and minimizing frequency drifts. In this embodiment the orifice 38 is configured to be a safety feature to maintain the frequency of operation of the pulse generator 18 within a prescribed range in case of failure of the needle valve 36, in particular to limit the maximum frequency. In addition the volume of chamber 32 can be changed by adjusting the control shaft of the needle valve. Doing so will change the pulse frequency of the pulse generator.

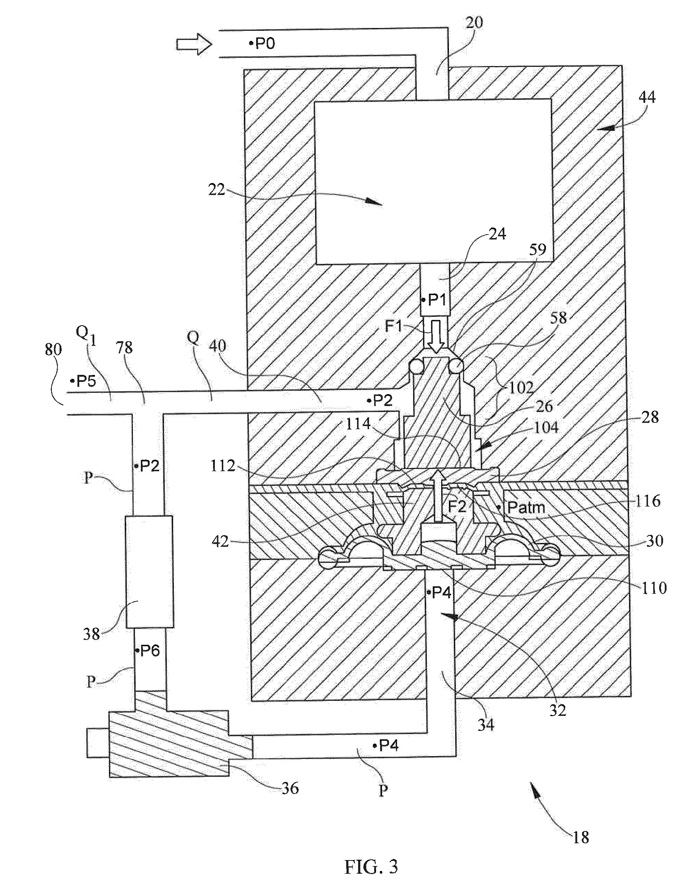

FIGS. 3-6 show the operation of the pulse generator 18. As shown in FIG. 3 compressed gases flows through inlet port 20 and into the AVC 22. The compressed gases apply a force F1 of magnitude shown by Equation 1 in a direction shown in FIG. 3 tending to deform first diaphragm 28. P1 is the pressure acting on plunger 26, and A1 is the area over which P1 acts when O-ring 58 is seated on O-ring seat 59.

$$F1 = P1 \times A1 \qquad \qquad \ldots$$

Equation 1

In the embodiment shown in FIG. 3, when force F1 overcomes force F2 described by Equation 2, the valve stem 26 moves downwardly and deforms first diaphragm 28. The weight of the valve stem 26 and of the valve button 42 have been neglected in this embodiment, but in another embodiment the orientation of the pulse generator with respect to the gravitational force is taken into account. In this embodiment as the valve stem 26 moves downwardly compressed gas from chamber 22 flows past the O-ring seat and through to outlet port 40. The fact that the valve stem has moved exposes an additional area (A2 in addition to A1) of valve stem 26 to pressure P1 exerted by the gas flowing past the O-ring seat. The exposure of a greater area (A1+A2 instead of A1) to the pressurized gas increases the magnitude of force F1 acting against F2, further moving the valve stem 26 in the direction of F1. In another embodiment the projected area exposed to pressurized gas as the valve stem 26 deforms first diaphragm 28 and allows compressed gas around the valve stem 26 and through to outlet port 40 remains A1 rather than increasing to A1 + A2.

$$F2 = P_{atm} \times A3 + KA \times DA \qquad \qquad \ldots$$

Equation 2

In this embodiment shown in FIGS. 2-8B the space between first diaphragm 28 and second diaphragm 30 is exposed to atmospheric pressure $P_{atm}$ while in other embodiments this space may be pressurized to a higher pressure and that pressure would be used for calculation of F2 in Equation 2 above instead of $P_{atm}$. In Equation 2 above A3 is the area of first diaphragm 28 exposed to atmospheric pressure. In this embodiment A3 is the projected area in the direction of force F2. In Equation 2 above, KA is the stiffness co-efficient (i.e. the spring constant) of first diaphragm 28 while DA is the deflection of the first diaphragm upon application of force by the valve stem 26. First diaphragm 28 is a linear spring in this embodiment while in another embodiment first diaphragm 28 is a non-linear spring. When F1 exceeds F2 the valve stem 26 moves in the direction of F1 establishing a fluidic connection between plunger port 24 and outlet port 40.

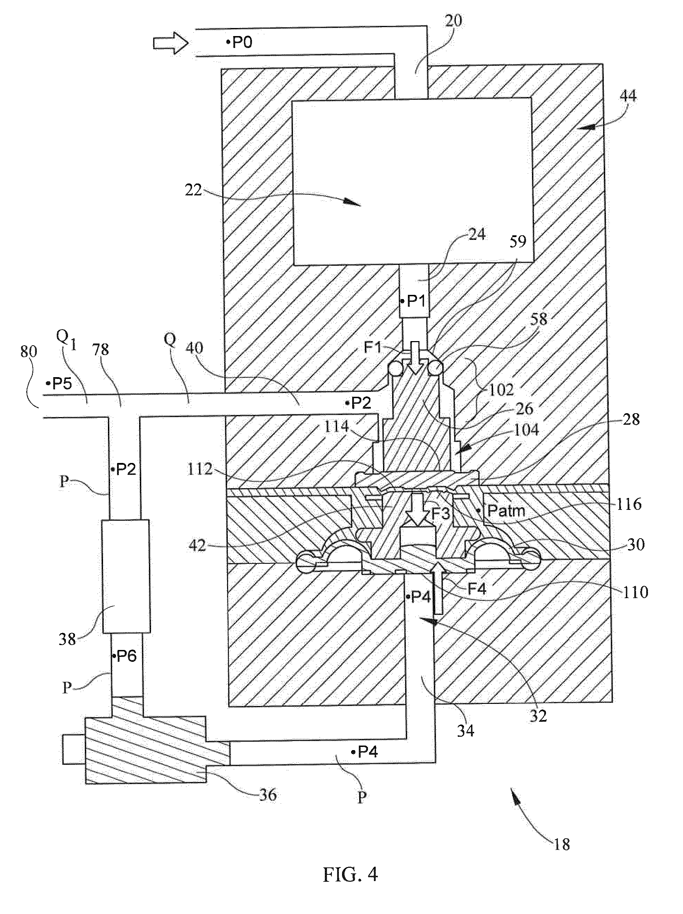

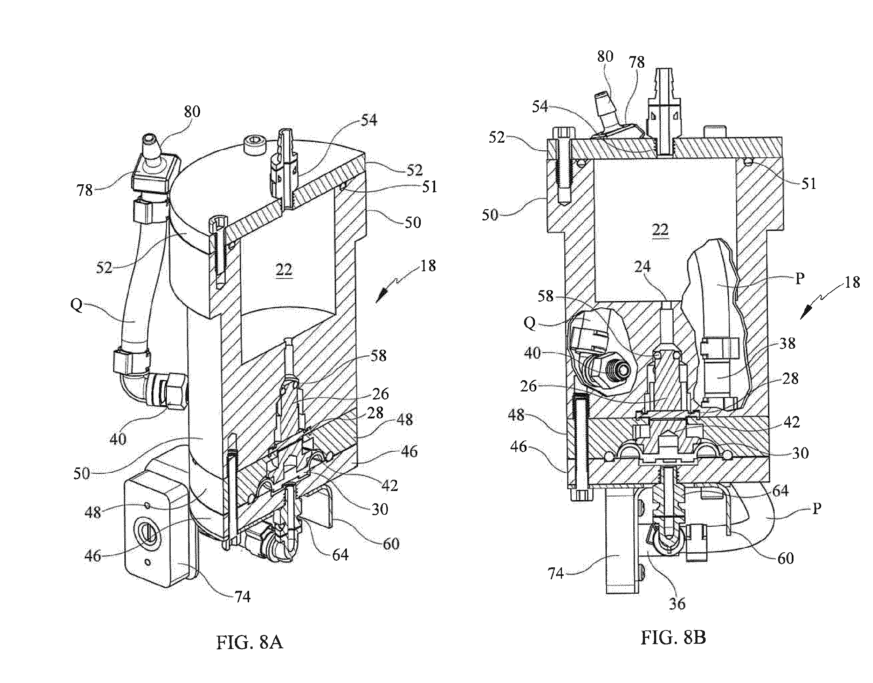

The pulse generator includes a conduit Q and a conduit P. Collectively the conduits Q and P define a fluid channel. The pulse generator also includes a patient delivery conduit Q1. Q1 connects with the fluid channel defined by Q and P at a juncture. The juncture is in the form of a Wye fitting 78 (FIGS. 7, 8A, 8B). As shown in FIG. 4 gases discharging from outlet port 40 flow through conduit Q. A portion of the gases flow through conduit Q1 to be delivered to a patient. Another portion of the gases flow through conduit P which includes orifice 38, and the needle valve 36 (conduits Q and P comprise a fluid channel extending from the outlet port to the input side of the diaphragm assembly). This increases the pressure P4 in second chamber 32. When force F4 due to pressure P4 acting on area A5 shown by Equation 4 exceeds force F3 shown by Equation 3, the valve button 42 moves upwards towards first diaphragm 28. Equation 3 below shows the summation of atmospheric pressure acting on area A4 in this embodiment and force required to deflect second diaphragm 30 where KB is the spring co-efficient (spring constant) of the second diaphragm and DB is its deflection. Second diaphragm 30 is configured to be a linear spring in this embodiment while in another embodiment second diaphragm 30 is a non-linear spring. In this embodiment clearance C is present between valve button 42 and first diaphragm 28.

$$F3 = P_{atm} \times A4 + KB \times DB \qquad \qquad \ldots \text{ Equation 3}$$

$$F4 = P4 \times A5 \qquad \qquad \ldots \text{ Equation 4}$$

where A4 is the area on top of diaphragm 30 exposed to $P_{atm}$ and A5 is the area on the bottom of diaphragm 30 exposed to P4.

Once valve button 42 contacts first diaphragm 28, further upward motion (or motion in direction of force F4) of valve button 42 urges upward displacement of first diaphragm 28. This, in turn, moves valve stem 26 in the direction of F4 (neglecting compression of first diaphragm 28 and weights of components). When force F4 exceeds force F3' shown below in equation 6 the valve button 42 pushes the valve stem 26 in the direction of F4 so as to reduce fluidic communication between plunger port 24 and outlet port 40. In another embodiment, no clearance is present between the valve button 42 and the first diaphragm 28 so that the valve button 42 displaces first diaphragm 28 if F4 exceeds F3' calculated by Equations 5 and 6.

$$F1' = [P2 \times (A1+A2)] - [P_{atm} \times A3 + KA \times DA] \qquad \ldots \text{Equation 5}$$

$$F3' = P_{atm} \times A4 + F1' + KB \times DB \qquad \ldots \text{Equation 6}$$

In equation 5 above F1' is the net force acting in the direction of F3 when the valve seat 26 is open wherein pressure P2 acts on a total projected area A1+A2. Force F3' acts in the direction of F3 in one embodiment.

When the valve stem 26 moves upwards to limit fluidic communication between plunger port 24 and outlet port 40, pressure at outlet port 40 drops and consequently pressure P4 in second chamber 32 decays. This reduction in P4 results in a reduction in force F4 acting on second diaphragm 30. When F4 diminishes to a value lower than F3 the valve button 42 begins to move in the direction of F3 and separates from first diaphragm 28.

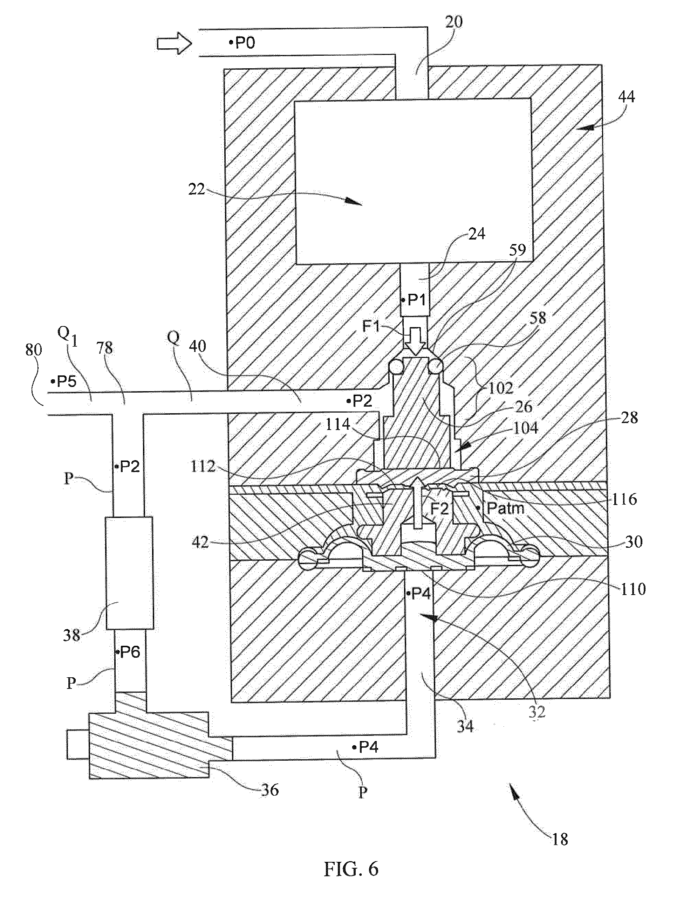

As shown in FIG. 6 the cycle repeats as valve stem 26 begins to move in the direction of F1 again when F1 exceeds F2. This opening and closing of the fluidic communication pathway between plunger port 24 and outlet port 40 creates oscillating pressure at 80 which is supplied for high frequency oscillation therapy.

FIG. 7 shows an exploded view of a pulse generator 18 for use with a system 10 to mobilize lung secretions. Although a particular design configuration is shown in FIG. 7 addition or removal of any combination of parts including but not limited to tubing, seals and valves are contemplated in other embodiments. Further aggregation of separate components shown in FIG. 7 is contemplated in other embodiments, in one exemplary embodiment portions of the body 44 may be integrated into a single piece.

As shown in FIG. 7 a fitting 54 allows air into the body 44. In this embodiment the body 44 comprises the valve base 46, first valve section 48, second valve section 50 and third valve section 52. The fitting 54 is configured to connect to third valve section 52. Third valve section 52 is configured to be connected to second valve section 50 by at least one fastener 56. An O-ring seal 51 seals between the second valve section 50 and third section 52. O-Ring 58, valve stem 26 and diaphragm 28 are captured between the second valve section 50 and first valve section 48. Valve button 42 and second diaphragm 30 are captured between the valve base 46 and first diaphragm 28. A bracket 60 is configured to mount to the valve base 46. Fasteners 56 are configured to connect the bracket 60, valve base 46, and first valve section 48 with second valve section 50. Needle valve 36 is configured to fluidly connect with valve base 46 via tube 68, clamp 66 and swivel elbow 64. The swivel elbow is mounted to the valve base 46. In this embodiment the needle valve 36 is configured to mount on the bracket 60 by fasteners 56 connecting with a locking sleeve 74. The connection includes an elastomeric bushing 72. An orifice 38 fluidly connects with the needle valve 36 at one end and the other end of the orifice 38 connects with a Wye fitting 78 via tube 68. Wye fitting 78 is also fluidly connected to outlet port 40 as shown in FIG. 2. Wye fitting includes a discharge leg 80. Wye fitting 78 supplies pulses of compressed gas through leg 80 for therapy.

FIG. 8 A & FIG. 8 B are perspective and front cross sectional views respectively of one embodiment of a pulse generator 18 for use with a system 10 to mobilize lung secretions showing some of the components shown in FIG. 7.

In another embodiment the AVC 22 is not incorporated into the body 44 and body 44 comprises a straight conduit up to plunger port 24.

During operation the pulse generator receives pressurized gas from a source thereof by way of inlet port 20. The pressurized gas moves the plunger 26 in a first direction toward the diaphragm assembly thereby increasing flow of the pressurized gas between the plunger port 24 and the outlet port 40. A first portion of the pressurized gas admitted through the outlet port acts on the input side 110 of the diaphragm assembly thereby urging the diaphragm assembly to move in a second direction opposite to the first direction. Pressure on the input side of the diaphragm assembly increases over time and eventually reverses the movement of the plunger so that the plunger moves in the second direction. The reverse movement of the plunger continues until O-ring 58 seats in seat 59 and no pressurized gas flows between the plunger port and the outlet port. The pressure acting on the input side of the diaphragm assembly then decays so that the pressurized gas can once again move the plunger in the first direction. In other words the above described behavior comprises a cycle which can repeats N times where N is greater than one. During each cycle a second portion of the pressurized gas flows into the patient delivery channel in a pulsating fashion.

An associated method of providing oscillatory pressurized gas comprises A) providing a pressurized gas from a source thereof, B) employing the pressurized gas to open a path to a fluid channel, C) directing a first portion of the gas to a delivery conduit, D) reducing the pressure of a second portion of the gas, and E) using the second portion to counteract the employing step. The step of using the second portion comprises accumulating the reduced pressure gas thereby elevating its pressure and enhancing its counteractiveness. Method steps B, C, D and E comprise a cycle. The method may proceed for N repetitions of the cycle where N is greater than one.

The foregoing description is for the purpose of illustration only. The use of any and all examples, or exemplary language (e.g., "such as") provided herein, is intended merely to better illustrate the subject matter. The use of the term "based on" and other like phrases indicating a condition for bringing about a result, is not intended to foreclose any other conditions that bring about that result. No language in the specification should be construed as indicating any element as essential.

Preferred embodiments are described herein, including the best mode known to the inventor. Of course, variations of those preferred embodiments will become apparent to those of ordinary skill in the art upon reading the foregoing description. The inventor expects skilled artisans to employ such variations as appropriate. Moreover, any combination of the above-described elements in all possible variations thereof is encompassed unless otherwise indicated herein or otherwise clearly contradicted by context.

**Claims**

1. A pulse generator for use with a system for mobilizing lung secretions comprising:

   a generator body;
   a plunger disposed inside the body, the plunger having a first end and a second end and being moveable to regulate fluid flow between a plunger port and an outlet port;
   a diaphragm assembly having an input side and an output side;
   a first diaphragm between the second end of the plunger and the diaphragm assembly, the first diaphragm having a plunger side facing the plunger and an opposite side facing the diaphragm assembly;
   a fluid channel extending from the outlet port to the input side of the diaphragm assembly;
   an orifice and a valve residing in the fluid channel.

2. The pulse generator of claim 1 comprising a body inlet port and a chamber between the inlet port and the plunger port.

3. The pulse generator of either claim 1 or claim 2 including a patient delivery conduit that forms a juncture with the fluid channel and wherein the orifice and valve are between the juncture and the input side of the diaphragm assembly.

4. The pulse generator of any proceeding claim wherein the valve is a needle valve.

5. The pulse generator of any proceeding claim wherein the diaphragm assembly comprises a second diaphragm and a button which defines at least part of the output side of the diaphragm assembly.

6. The pulse generator of any proceeding claim wherein the first diaphragm and the diaphragm assembly are separated by a clearance space.

7. The pulse generator of any proceeding claim wherein during operation:

   A) the pulse generator receives pressurized gas;
   B) the pressurized gas moves the plunger in a first direction toward the diaphragm assembly thereby increasing flow of the pressurized gas between the plunger port and the outlet port;
   C) a first portion of the pressurized gas admitted through the outlet port exerts pressure on the input side of the diaphragm assembly thereby generating a force which acts in a second direction which is opposite to the first direction; and
   D) pressure on the input side of the diaphragm assembly increases over time and reverses the movement of the plunger so that the plunger moves in the second direction thereby decreasing flow of the pressurized gas between the plunger port and the outlet port.

8. The pulse generator of claim 7 wherein during operation:

E) the reverse movement of the plunger continues until no pressurized gas flows between the plunger port and the outlet port.

9. The pulse generator of claim **8** wherein operations **B, C, D** and **E** comprise a cycle and the cycle repeats **N** times where **N** is greater than one.

10. The pulse generator of any one of claims 7 to 9 including a patient delivery conduit that forms a juncture with the fluid channel and wherein a second portion of the pressurized gas flows into the patient delivery channel in a pulsating fashion.

11. The pulse generator of any proceeding claim wherein the valve and orifice are external to the valve body.

12. A method of providing oscillatory pressurized gas comprising:

    **A)** providing a pressurized gas from a source thereof;
    **B)** employing the pressurized gas to open a path to a fluid channel;
    **C)** directing a first portion of the gas to a delivery conduit;
    **D)** reducing the pressure of a second portion of the gas; and
    **E)** using the second portion to counteract the employing step.

13. The method of claim **12** wherein the step of using the second portion comprises accumulating the reduced pressure gas thereby elevating its pressure over time and enhancing its counteractiveness.

14. The method of claim **12** wherein steps **B, C, D** and **E** comprise a cycle and wherein the method includes **N** repetitions of the cycle where **N** is greater than one.

15. The method of claim **13** wherein steps **B**, **C, D** and **E** comprise a cycle and wherein the method includes **N** repetitions of the cycle where **N** is greater than one.

FIG. 1

FIG. 2

FIG. 3

FIG. 4

FIG. 5

FIG. 6

FIG. 7

FIG. 8B

FIG. 8A